# EUROPEAN PATENT APPLICATION

(11) **EP 2 530 068 A1**
(43) Date of publication of application: **05.12.2012**
(21) Application number: 11382178.9
(22) Date of filing: 31.05.2011
(51) Int. Cl.: C07C 55/02, C07F 3/00, A61K 31/194, A61P 19/10

(54) **New strontium salts, synthesis and use thereof in the treatment of osteoporosis**

(71) Applicant: LACER, S.A., 08025 Barcelona (ES)
(72) Inventor: Mourelle Mancini, Marisabel, E-08028 Barcelona (ES); Del Castillo Nieto, Juan Carlos, E-08025 Barcelona (ES)
(74) Representative: ABG Patentes, S.L.

(57) **Abstract**

The present invention relates to a strontium salt (I) 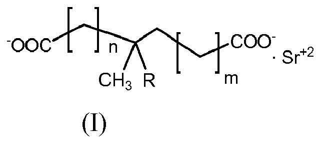 wherein
n is an integer which can take the values of 0 or 1
m is an integer which can take the values of 0 or 1
R is hydrogen, methyl or ethyl

and stereoisomers or hydrates thereof, to methods for its synthesis, and use in the treatment and/or prophylaxis of a cartilage and/or bone disease and/or conditions resulting in a dysregulation of cartilage and/or bone metabolism in a mammal.

## Description

### FIELD OF THE INVENTION

The present invention relates to new compounds, their use for the treatment of osteoporosis, and synthesis thereof.

### BACKGROUND OF THE INVENTION

The use of strontium salts for use in the treatment or prophylasis of bone and/or cartilage conditions such as osteoporosis and other diseases and conditions affecting structural integrity of bone is know. As an example WO 2004/098619 A2 teaches that strontium salts have prophylactic and/or therapeutic value in the treatment and/or prophylaxis of a cartilage and/or bone disease and/or conditions resulting in a dysregulation of cartilage and/or bone metabolism in a mammal, such as, e. g. , a human female or male adult, adolescent or a child, such as, e. g., osteoporosis, osteoarthritis, osteopetrosis, osteopenia and Paget's disease, hypercalcemia of malignancy, periodontal disease, hyperparathyroidism, periarticular erosions in rheumatoid arthritis, osteodystrophy, myositis ossificans, Bechterew's disease, malignant hypercalcemia, osteolytic lesions produced by bone metastasis, bone pain due to bone metastasis, bone loss due to sex steroid hormone deficiency, bone abnormalities due to steroid hormone treatment, bone abnormalities caused by cancer therapeutics, osteomalacia, Bechet's disease, hyperostosis, metastatic bone disease, immobilization-induced osteopenia or osteoporosis, or glucocorticoid-induced osteopenia or osteoporosis, osteoporosis pseudoglioma syndrome, idiopathic juvenile osteoporosis, for the improvement of fracture healing after traumatic or atraumatic fracture, and for the maintenance or increase of energy level, for building up or strengthening muscle tissues and for weight gain. EP 0 415 850 A1 discloses divalent salts of ranelic acid and more particularly strontium ranelate of formula: 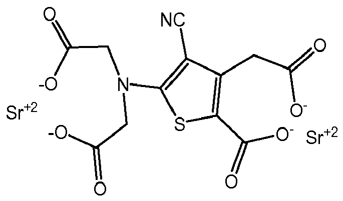 and its use for the treatment of bone disorders, cutaneous and vascular ageing, hepatic disorders and dental disorders. Strontium ranelate is approved for use in the treatment of osteoporosis in postmenopausal women to reduce the risk of vertebral and hip fractures. The bioavailability of strontium ranelate has been reported to be low (in the range from 19% to 27%) (European Medicines Agency. Protelos: summary of product characteristics).

Strontium ranelate is the only strontium salt approved as a medicament for the treatment of osteoporosis although clinical trials are under way with other salts such as strontium malonate.

Strontium citrate is also commercially available as a dietary supplement in the United States and Canada and the patent literature discloses many other strontium salts.

A good example is WO 2004/098617 A2 in the name of Nordic Bone A/S (later transferred to Osteologix A/S) which discloses controlled release pharmaceutical compositions comprising strontium salts for the treatment of diseases and condition affecting metabolism and/or structural integrity of cartilage and/or bone. The application discloses that it is preferred to formulate compositions for administration once daily comprising strontium salts having a relatively low water solubility under physiological conditions (i.e. solubility below 1 g/l at 40°C). The document also contains a long list of explicitly mentioned strontium salts although the bioavailability is only provided for strontium chloride, strontium ranelate and strontium citrate and the data provided therein show than strontium citrate has a greater bioavailability (AUC) than strontium ranelate. Other documents disclosing strontium salts for use as a medicament are EP 0 349 432 B1, EP 1 136 065 B1, EP 0 737 478 B1, EP 0 737 471 A2, EP 0 813 869 B1, WO 98/35657 A, WO 2003/028742 A, EP 1 741 436 A, EP 1 560 578 B1, WO 2004/084920 A, WO 2004/098618 A, WO 2004/098619 A, EP 1 745 791 A, EP 1 523 985 B, EP 1 682 156 B, WO 2005/082385, WO 2005/108339 A, WO 2005/123098 A2, WO 2005/123192 A2, WO 2005/123193 A2, EP 1 768 748 B1, EP 1 642 897 B9, WO 2006/089546 A1, WO 2007/003200 A2, WO 2007/149815 A1, WO 2007/149816 A2, EP 2 067 478 A1, WO 2008/061535 A1 and WO 2010/117346 A2.

There has been a continuous interest in finding strontium salts with a bioavailability higher than that of strontium ranelate and strontium citrate is a good example of such a salt. Nevertheless, there is still room for improving the bioavailability of strontium salts beyond that of strontium citrate and strontium ranelate.

Accordingly, there is a need to find new pharmaceutically acceptable strontium salts having an improved bioavailability.

### SUMMARY OF THE INVENTION

The present invention provides strontium salts with an improved bioavailability.

Accordingly, in a first aspect the present invention is directed to strontium salts of formula (I), stereoisomers or hydrates thereof (compounds of the invention) 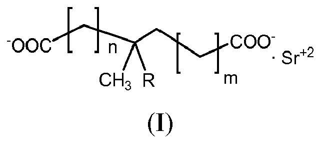 wherein
n is an integer which can take the values of 0 or 1
m is an integer which can take the values of 0 or 1
R is hydrogen, methyl or ethyl.
and stereoisomers or hydrates thereof.

A second aspect of the invention relates to a method for the synthesis of the compounds of the invention, wherein an aqueous solution of a dicarboxylic acid of formula (II): 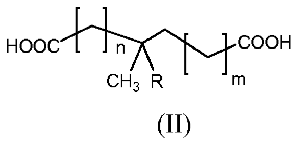 is reacted with strontium hydroxide, maintained at a temperature in the range of 80-100°C for a period in the range of 30 min to 6 hours, filtered, cooled to room temperature and added acetone to precipitate the salt of formula (I).

A further aspect is a compound of the invention for use as a medicament.

A further aspect is a compound of the invention for use in the treatment of osteoporosis.

A further aspect is the use of a compound of the invention for the preparation of a medicament for the treatment of and/or prophylaxis of a cartilage and/or bone disease and/or conditions resulting in a dysregulation of cartilage and/or bone metabolism in a mammal, such as, e. g. , a human female or male adult, adolescent or a child, such as, e. g. , osteoporosis, osteoarthritis, osteopetrosis, osteopenia and Paget's disease, hypercalcemia of malignancy, periodontal disease, hyperparathyroidism, periarticular erosions in rheumatoid arthritis, osteodystrophy, myositis ossificans, Bechterew's disease, malignant hypercalcemia, osteolytic lesions produced by bone metastasis, bone pain due to bone metastasis, bone loss due to sex steroid hormone deficiency, bone abnormalities due to steroid hormone treatment, bone abnormalities caused by cancer therapeutics, osteomalacia, Bechet's disease, hyperostosis, metastatic bone disease, immobilizationinduced osteopenia or osteoporosis, or glucocorticoid-induced osteopenia or osteoporosis, osteoporosis pseudoglioma syndrome, idiopathic juvenile osteoporosis, for the improvement of fracture healing after traumatic or atraumatic fracture, and for the maintenance or increase of energy level, for building up or strengthening muscle tissues and for weight gain.

A further aspect is a method of treatment and/or prophylaxis of a cartilage and/or bone disease and/or conditions resulting in a dysregulation of cartilage and/or bone metabolism in a mammal, such as, e. g. , a human female or male adult, adolescent or a child, such as, e. g. , osteoporosis, osteoarthritis, osteopetrosis, osteopenia and Paget's disease, hypercalcemia of malignancy, periodontal disease, hyperparathyroidism, periarticular erosions in rheumatoid arthritis, osteodystrophy, myositis ossificans, Bechterew's disease, malignant hypercalcemia, osteolytic lesions produced by bone metastasis, bone pain due to bone metastasis, bone loss due to sex steroid hormone deficiency, bone abnormalities due to steroid hormone treatment, bone abnormalities caused by cancer therapeutics, osteomalacia, Bechet's disease, hyperostosis, metastatic bone disease, immobilization-induced osteopenia or osteoporosis, or glucocorticoid-induced osteopenia or osteoporosis, osteoporosis pseudoglioma syndrome, idiopathic juvenile osteoporosis, for the improvement of fracture healing after traumatic or atraumatic fracture, and for the maintenance or increase of energy level, for building up or strengthening muscle tissues and for weight gain, which method comprises administering to a patient in need of such a treatment a therapeutically effective amount of at least one compound of formula (I) or a pharmaceutical composition thereof.

A further aspect is directed to a pharmaceutical composition comprising a compound of the invention and a pharmaceutically acceptable carrier.

A further aspect is directed to a pharmaceutical composition comprising a compound of the invention, a pharmaceutically acceptable carrier and one or more additional drugs known to be effective in the treatment of osteoporosis such as vitamin D; estrogen analogs useful in estrogen replacement therapy such as estradiol and conjugate equine estrogens (CEE); parathyroid hormone and its analogs such as Teriparatide and PTH-131A; selective estrogen receptor modulators (SERMs) such as Raloxifene, Lasofoxifene, Arzoxifene and Bazedoxifene; selective tissue estrogenic activity regulators such as Tibolone; bisphophonates such as Zoledronate, Risedronate, Ibandronate, Alendronate, Olpadronate, Neridronate, Pamidronate, Etidronate, Clodronate, Tiludronate; Calcitonines such as salcitonine; sodium fluoride; cathepsin K inhibitors such as Odanacatib and ONO-5334; antagonists for the receptor activator of nuclear factor kappa B ligand (RANKL) such as Osteoprotegerin and monoclonal antibodies targeting RANKL such as Denosumab; 3-hydroxy-3-methyl-glutaryl-CoA (HMG-CoA) reductase inhibitors such as Atorvastatin, Cerivastatin, Fluvastatin, Lovastatin, Mevastatin, Pitavastatin, Pravastatin, Rosuvastatin, Simvastatin and other strontium salts such as strontium citrate, strontium malonate and strontium ranelate.

Due to the intrinsic alkaline properties of strontium ion, strontium salts elevate the pH when solubilised in aqueous media, such as the gastric juice of the stomach, thereby providing a gastrointestinal protective effect. Thus, when administered in combination with other drugs, such as bisphosphonates, NSAIDs, cytostatic agents or glucocorticoids which are known to be associated with significant gastro-intestinal (G1) adverse events, the strontium salts of the present invention will have a beneficial effect and serve to prevent or reduce occurrence of gastrointestinal adverse events.

Thus, in a further aspect of the present invention the strontium salts of the present invention could be used in association with drugs, which are known to produce gastro-intestinal (G1) adverse events, to reduce the incidence of such adverse events. In such a use the strontium salts could also be used in association with other stomach protecting agents. Examples of stomach protecting agents are histamine H2-receptor antagonists such as Cimetidine, Ranitidine, Famotidine and Nizatidine; proton pump inhibitors such as Omeprazole, Lansoprazole, Dexlansoprazole, Esomeprazole, Pantoprazole and Rabeprazole or with agents that increase gastric mucus.

The compounds of the invention have shown higher bioavailability than strontium salts of the prior art. As a consequence, reduced dosages are needed which in turn may help provide less side effects.

### DETAILED DESCRIPTION OF THE INVENTION

The compounds of the present invention are strontium salts of formula (I), stereoisomers or hydrates thereof (compounds of the invention) 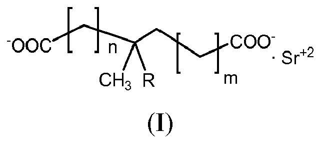 wherein
n is an integer which can take the values of 0 or 1
m is an integer which can take the values of 0 or 1
R is hydrogen, methyl or ethyl.
and stereoisomers or hydrates thereof.

The term "hydrate" according to this invention is to be understood as meaning any form of the salts according to the invention which has water molecules attached to it via non-covalent bonding.

It will be immediately apparent to the skilled person that the present invention encompasses all possible stereoisomers of the compounds described herein. An stereoisomer is understood by the skilled person as compounds made up of the same atoms bonded by the same sequence of bonds but having different three-dimensional structures which are not interchangeable, e.g. diastereoisomers or enantiomers. Separation methods of the different diastereoisomers or enantiomers by chemical and/or physical means are readily available to the skilled person. For example, an enantiomerically enriched mixture of enantiomers may be obtained at any stage of the synthesis by purification under chiral conditions, such as chiral supercritical fluid chromatography. Following the same methods pure enantiomers may also be isolated.

### Synthesis of the salts of formula (I)

As mentioned above, the salts of the invention may be prepared by reaction of an aqueous solution of a dicarboxylic acid of formula (II): 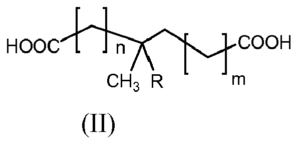 with strontium hydroxide, maintaining the reaction media at a temperature in the range of 80-100°C for a period in the range of 30 min to 6 hours, filtering the resulting reaction media, cooling the filtered solution to room temperature and adding acetone to precipitate the salt of formula (I).

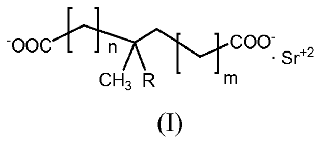

In an embodiment of the invention the molar ratio of dicarboxylic acid (II) to strontium hydroxide used for the preparation of the compounds of formula (I) ranges from 0.60 to 1.67, preferably from 0.8 to 1.25, more preferably from 0.9 to 1.1 and still more preferably from 0.99 to 1.01.

The dicarboxylic acids of formula (II) are commercially available or may be prepared by methods described in the literature.

According to an embodiment of the invention in the compounds of formula (I) n is an integer which can take the values of 0 or 1, m is an integer which can take the values of 0 or 1, and R is hydrogen or methyl defining compounds of formula (Ia): 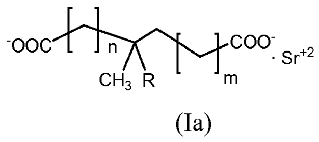 and stereoisomers or hydrates thereof.

According to another embodiment of the invention in the compounds of formula (I) n is an integer which can take the values of 0 or 1, m is an integer with a value of 0 and R is hydrogen or methyl defining compounds of formula (Ib): 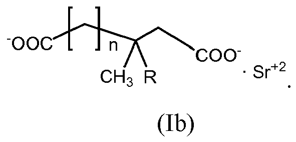 and stereoisomers or hydrates thereof.

According to still another embodiment of the invention in the compounds of formula (I) n is an integer with a value of 1, m is an integer with a value of 0 and R is hydrogen defining the compound of formula (Ic) (strontium 3-methylpentanedioate): 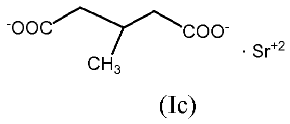 and hydrates thereof.

According to an embodiment, the compounds of the invention are selected from the group consisting of:
strontium (R)-3-methylhexanedioate hemihydrate
strontium 3,3-dimethylpentanedioate monohydrate
strontium 2,2-dimethylsuccinate monohydrate
strontium 3-methylpentanedioate
strontium 2-methylpentanedioate hemihydrate
strontium 3-ethyl-3-methylpentanedioate

### Uses of the salts of the invention

According to a further aspect, the present invention is directed to a method of treatment and/or prophylaxis of a cartilage and/or bone disease and/or conditions resulting in a dysregulation of cartilage and/or bone metabolism in a mammal, such as, e. g. , a human female or male adult, adolescent or a child, such as, e. g. , osteoporosis, osteoarthritis, osteopetrosis, osteopenia and Paget's disease, hypercalcemia of malignancy, periodontal disease, hyperparathyroidism, periarticular erosions in rheumatoid arthritis, osteodystrophy, myositis ossificans, Bechterew's disease, malignant hypercalcemia, osteolytic lesions produced by bone metastasis, bone pain due to bone metastasis, bone loss due to sex steroid hormone deficiency, bone abnormalities due to steroid hormone treatment, bone abnormalities caused by cancer therapeutics, osteomalacia, Bechet's disease, hyperostosis, metastatic bone disease, immobilizationinduced osteopenia or osteoporosis, or glucocorticoid-induced osteopenia or osteoporosis, osteoporosis pseudoglioma syndrome, idiopathic juvenile osteoporosis, for the improvement of fracture healing after traumatic or atraumatic fracture, and for the maintenance or increase of energy level, for building up or strengthening muscle tissues and for weight gain, comprising the administration of a therapeutically effective amount of the compounds of formula (I).

The term "treatment" in the context of this specification means administration of a compound or formulation of the invention to eliminate or ameliorate anyone of the above mentioned conditions, or symptoms associated to these conditions.

The term "prophylaxis" in the context of this specification means administration of a compound or formulation of the invention to prevent the occurrence of anyone of the above mentioned conditions, or symptoms associated to these conditions.

It is an embodiment of the present invention to use the compounds of formula (I) in the treatment and/or prophylaxis of osteoporosis, osteopenia, artrosis and osteoarthritis, preferably in the treatment and/or prophylaxis of osteoporosis.

According to a further aspect, the present invention is directed to a pharmaceutical composition comprising a compound of the invention and a pharmaceutically acceptable carrier.

The term "carrier" refers to a diluent, adjuvant, excipient, or vehicle with which the active ingredient is administered. Such pharmaceutical carriers can be sterile liquids, such as water and oils, including those of petroleum, animal, vegetable or synthetic origin, such as peanut oil, soybean oil, mineral oil, sesame oil and the like. Water or aqueous solution saline solutions and aqueous dextrose and glycerol solutions are preferably employed as carriers, particularly for injectable solutions, also buffers, isotonic agents or agents capable increasing solubility. Suitable pharmaceutical carriers are described in "Remington's Pharmaceutical Sciences" by E.W. Martin or "Tratado de Farmacia Galénica", C. Faulí i Trillo, Luzán 5, S.A. de Ediciones, 1993.

The pharmaceutical composition of the invention may be administered in the form of different preparations. Non limiting examples are preparations for oral administration, e.g. tablets, capsules, syrups or suspensions. The compounds of the invention are preferably administered orally.

Generally an effective administered amount of a compound used in the invention will depend on the relative efficacy of the compound chosen, the severity of the disorder being treated, or the age, weight or mode of administration. However, active compounds will typically be administered once or more times a day, for example 1, 2, 3 or 4 times daily, with typical total daily doses in the range of from 0.01 to 100 mg/kg/day.

The compounds used in the present invention may also be administered with other drugs to provide a combination therapy. The other drugs may form part of the same composition, or be provided as a separate composition for administration at the same time or at different time.

### Experimental section

The examples set forth in this description detail the suitable processes for obtaining several compounds which can be assigned to formula (I). In view of said examples, it is evident and direct for a person skilled in the art to obtain the compounds which are not explicitly exemplified, by means of applying modifications of the methods set forth, characteristic of the common general knowledge of the persons skilled in the art.

Consequently, the examples set forth below must not be interpreted as limiting the scope of the present invention, but rather as an additional and more detailed explanation which guides the person skilled in the art to a deeper understanding of the invention.

The following abbreviations are used in the following examples:
- D₂O: Deuterium oxide
- EtOH: Ethanol
- HPLC: high pressure liquid chromatography
- ICP-MS: Inductively coupled plasma mass spectrometry
- F-AAS: Flame Atomic Absorption Spectroscopy
- ¹³C-NMR: Carbon 13 Nuclear Magnetic Resonance
- ¹H-NMR: Proton Nuclear Magnetic Resonance
- NIST: National Institute of Standards and Technology
- MW: Molecular weight
- RT: Room temperature
- TLC: Thin layer chromatography
- TGA: Thermogravimetric analysis
- e.e.: Enantiomeric Excess

### NUCLEAR MAGNETIC RESONANCE

The nuclear magnetic resonance spectra have been carried out in a Varian Gemini-200 apparatus.

The ¹H -NMR spectra indicate the working frequency and the solvent used to make the spectrum. The position of the signals is indicated in δ (ppm), using the signal of the protons of the solvent as reference. The reference values are 4.46 ppm for D₂O. Within brackets are indicated the number of protons corresponding to each signal measured by electronic integration and the type of signal using the following abbreviations: s (singlet), d (doublet), t (triplet), q (quadruplet), s.b. (signal broad). The signal assignation is indicated in some cases.

The ¹³C-NMR spectra indicate the working frequency and the solvent used to make the spectrum. The position of the signals is indicated in δ (ppm), using the central signal of the solvent as reference.

### ELEMENTAL ANALYSIS

The percentage of carbon (C), Nitrogen (N) and Hydrogen (H) were determined in a EA-1108 elemental analyzer from C.E. Instruments (Thermo Fisher Scientific) using the standard operating conditions recommended by the supplier of the apparatus and using atropine as a standard. Samples of approximately 1.000 mg of the product to be analyzed were weighted with a Metller Toledo MX5 micro balance.

The percentage of Strontium (Sr) was determined by graphite furnace atomic absorption spectrometry (GFAAS) using in a Varian Techtron model SpectrAA-400 with Zeeman background correction.

### THERMOGRAVIMETRY (TGA)

- Equipment:: Mettler TGA/SDTA851 Thermobalance
- Crucible:: Alumina 70µL
- Gas:: Dry nitrogen at 50 mL/min
- Method:: Heating from 30°C to 550°C at a rate of 10°C/min

### A) SYNTHESIS

### General method for preparation of crystalline salts by neutralization of dicarboxylic acids with strontium hydroxide.

A small amount of the proper organic dicarboxilic acid (0.02 mol) was dissolved in water (100ml). Then strontium hydroxide (Aldrich 433608) (0.02 mol) was added and stirred at 80-100°C during 3h.

The solution is filtered while hot on a Büchner funnel and the filtrate was allowed to cool at room temperature with stirring overnight. Then 150ml of acetone were added and the white precipitated solid was collected by filtration.

The solid was dried at 65°C under vacuum (< 1mm/Hg) in the presence of a draying agent such as Sicapent® (Merck 1.00543.2800) during 24h.

Following the method described above and using the corresponding dicarboxylic acids the following strontium salts were prepared.

### Example 1. Strontium (R)-3-methylhexanedioate hemihydrate

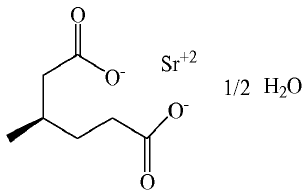

### Termogravimetric Analysis (TGA)

The product suffered a weight loss of 3.55% between 30.36°C and 168.97°C (as determined by TGA) corresponding to half molecule of water. Over 400°C decomposition is produced with a weight loss of 38.48%

**Elemental Analysis (C₇H₁₀O₄Sr½H₂O,MW: 254.8):**
**Calculated C**: 32.99% **H**:4.35% **Sr**: 34.38%
**ExperimentalC:** 32.41% **H**: 4.35% **Sr**: 34.00%
**¹H -RMN (D₂O, 200 MHz):** 4.66 (D₂O); 2.10-1.95 (m., 3H,); 1.83-1.50 (m, 2H,); 1.50-1.20 (m., 2H,); 0.71 (d., J=8 Hz, 3H, CH₃)
**¹³C-RMN (D₂O, 50 MHz):** 183.74 (1C, C=O); 182.92 (1C, C=O); 44.77 (1C, CH₂); 34.89 (1C, CH₂); 32.67 (1C, CH₂); 30.42 (1C, CH); 18.37 (1C, CH₃).

### Example 2. Strontium 3,3-dimethylpentanedioate monohydrate

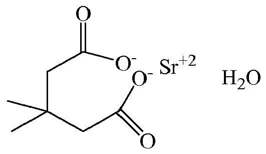

### Termogravimetric Analysis (TGA)

The product suffered a weight loss 5.18% between 30.36°C and 203.62°C (as determined by TGA) corresponding to a molecule of water. Over 400°C decomposition is produced with a weight loss of 37.25%

**Elemental Analysis (C₇H₁₂O₅Sr, MW: 263.8):**
**Calculated C**:31.87% **H**:4.59% **Sr**: 32.22%
**ExperimentalC:** 31.61% **H**:4.30% **Sr**: 34.10%
**¹H -RMN (D₂O, 200 MHz):** 4.66 (D₂O) 1.99 (s., 4H,); 0.89 (s., 6H, CH₃)
**¹³C-RMN (D₂O,** 50 **MHz):** 181.47 (2C, C=O); 50.59 (2C, CH₂); 31.57 (1C, C); 26.50 (2C, CH₃).

### Example 3. Strontium 2,2-dimethylsuccinate monohydrate

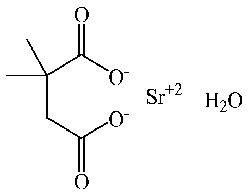

### Termogravimetric Analysis (TGA)

The product suffered a weight loss of 7.2% between 29.51°C and 179.45°C (as determined by TGA) corresponding to a molecule of water. Over 500°C decomposition is produced with a weight loss of 30.50%

**Elemental Analysis(C₆H₁₀O₅Sr, MW:** 249.8):
**Calculated C**:28.85% **H:**4.04% **Sr**: 35.08%
**ExperimentalC: 29.35% H**: **3.81% Sr**: **35.50%**
**¹H -RMN (D₂O, 200 MHz):** 4.65 (D₂O); 2.22 (s., 2H,); 0.99 (s., 6H, CH₃)
**¹³C-RMN (D₂O, 50 MHz):** 187.14 (1C, C=O); 181.09 (1C, C=O); 41.59 (1C, C); 40.06 (1C, CH₂); 24.99 (2C, CH₃).

### Example 4. Strontium 3-methylpentanedioate

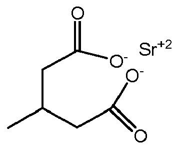

### Termogravimetric Analysis (TGA)

The product suffered a weight loss of 31.32% between 300.0°C and 550.0°C (as determined by TGA) corresponding to decomposition of the product

**Elemental Analysis (C₆H₈O₄Sr, MW: 231.7):**
**Calculated C:**31.10% **H**: 3.48% **Sr**: 37.81%
ExperimentalC: 29.67% **H**:3.11% **Sr**: 39.50%
**¹H -RMN (D₂O, 200 MHz):** 4.65 (D₂O); 2.04-1.78 (m., 5H,); 0.72 (d., J=3.2Hz, 3H, CH₃)
**¹³C-RMN (D₂O, 50 MHz):** 182.37 (2C, C=O); 44.83 (2C, CH₂); 28.81 (1C, CH); 18.67 (1C, CH₃).

### Example 5. (±) Strontium 2-methylpentanedioate hemihydrate

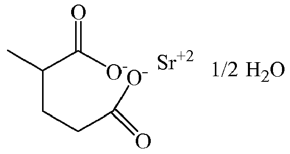

### Termogravimetric Analysis (TGA)

The product suffered a weight loss of 3.35% between 123.62°C and 191.68°C (as determined by TGA) corresponding to half molecule of water. Over 400°C decomposition is produced with a weight loss of 31.05%

**Elemental Analysis (C₆H₈O₄Sr ½ H₂O, MW: 240.7):**
**Calculated C:** 29.94% **H**: 3.77% **Sr**: 36.39%
**ExperimentalC:** 29.30% **H**: 3.72% **Sr**: 36.90%
**¹H -RMN (D₂O, 200 MHz):** 4.65 (D₂O) 2.20-1.94 (m., 3H,); 1.70-1.30 (m., 2H,); 0.89 (d.d, J=7Hz, J=2.6Hz, 3H, CH₃)
**¹³C-RMN (D₂O, 50 MHz):** 186.15 (1C, C=O); 183.14 (1C, C=O); 42.23 (1C, CH); 35.36 (1C, CH₂); 30.39 (1C, CH₂); 16.94 (1C, CH₃).

### Example 6. Strontium 3-ethyl-3-methylpentanedioate

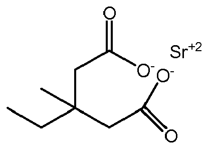

### Termogravimetric Analysis (TGA)

The product suffered a weight loss of 42.16% between 350.0°C and 550.0°C (as determined by TGA) corresponding to decomposition of the product

**Elemental Analysis (C₈H₁₂O₄Sr , MW: 259.8):**
**Calculated C:** 36.98% **H**:4.66% **Sr**: 33.73%
**ExperimentalC:** 37.33% **H:** 4.51% **Sr:** 33.50%
**¹H -RMN (D₂O, 200 MHz):** 4.65 (D₂O) 2.07 (s., 4H, 2 CH₂); 1.26 (q., J=7.2 Hz, 2H, CH₂); 0.86 (s, 3H, CH₃); 0.67 (t., J=7.2Hz, 3H, CH₃)
**¹³C-RMN (D₂O, 50 MHz):** 181.77 (2C, C=O); 47.50 (2C, CH₂); 34.47 (1C, C); 31.76 (1C, CH₂); 23.85 (1C, CH₃); 7.45 (1C, CH₃).

### B) PHARMACOLOGY

### Description of the pharmacological processes

### Pharmacokinetic assay of strontium salts in rats by the oral route

Bioavailability of the strontium salts of the invention was compared with that of prior art products through determination of the plasmatic concentration of strontium (ppm) after the oral administration of the salts to be tested. Blood samples were collected at selected intervals during a period of 24 hours and the strontium levels were plotted versus time. The area under the curve at 2 and 24 hours was then calculated.

The assay was carried out using male Wistar rats, supplied from Janvier (Francia) and weighting 125-150 g. The animals were placed in a ventilated box with filtered air (absolute filters) at a temperature of 21°C ± 3°C and a relative humidity of 55% ± 15%.

The ventilated box was placed in a room equipped with a ventilation system that changed room's air 10 times per hour. The room was illuminated during 12 hours a day and maintained dark the other 12 hours.

Rats wed fed with a global rodent diet based on compacted fodder ref. 2014 de Harlan (Spain). Animals had free access to drinking water.

The animals were fasted during the 5 hours preceding the oral administration of the compounds to be tested.

The compounds were dissolved in an aqueous solution/suspension of 1% Tween 80 and 0.5% carboxymethylcellulose and administered at a dose adjusted to provide 137 mg of strontium cation per kg of body weigth. The control group was administered only the aqueous solution of excipients (1% Tween 80 and 0.5% carboxymethylcellulose).

The excipient solution was prepared using demineralized water.

The compounds to be tested were solubilized/suspended in a volume corresponding to 10ml/kg of corporal weight. To maintain the compounds in an homogenous solution or suspension the formulations were homogenized in a vortex mixer immediately before administration.

At selected times, counting from the moment of the administration, animals were anesthetized with an intraperitoneal injection of 40 mg/kg sodium pentobarbital and blood samples were collected from all animals extracting them directly from the heart with a previously heparinized set of a needle (21G) and a syringe.

Samples were collected from 2 animals at each of the following times: 0 and 30 min and 1, 2, 3, 5, 6, 8 and 24 hours after administration of the strontium compounds solutions/suspensions.

The heparinized blood samples were centrifuged (10 min, 1.270 x g, +4°C). Blood plasma was separated and transferred to tubes that were kept in a refrigerator (+4°C) until their strontium content was later analyzed by graphite furnace atomic absorption spectrometry (GFAAS).

### ATOMIC ABSORPTION SPECTROSCOPY

### Sample preparation:

The samples of the product to be analyzed were homogenized in a vortex. Then they were transferred to a Teflon reactor, weighted and treated overnight with 2 ml of HNO₃ ("Baker Instra") at 90°C. 17 ml of deionized water (Purelab Ultra quality) were then added to the treated mixture.

### Strontium determination:

The determination of the percentage of strontium in the samples was made by graphite furnace atomic absorption spectrometry in a "Varian SpectrAA" apparatus equipped with a Zeeman corrector in standard conditions. Injection was triplicated.

The calibration of the apparatus was made using 4 solutions (10 - 25 - 50 - 100 mg/L) prepared by diluting a 1g/L standard solution.

### Results:

The following table shows for each compound the area under the curve (AUC) of the plasmatic levels vs. time for the periods of 0-2 hours and 0-24 hours.

| Example | AUC₀₋₂h (mg.h/l) | AUC₀₋₂₄ₕ (mg.h/l) |
|---|---|---|
| 1 | 55.6 | 216.6 |
| 2 | 41.3 | 212.0 |
| 3 | 58.8 | 241.0 |
| 4 | 65.2 | 299.8 |
| 5 | 56.2 | 215.1 |
| 6 | 44.3 | 196.5 |
| Strontium ranelate 7H₂O | 22.9 | 132.1 |
| Strontium citrate • 6H₂O | 23.6 | 165.7 |

## Claims

1. A strontium salt of formula (I) 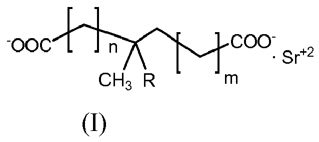 wherein
n is an integer which can take the values of 0 or 1
m is an integer which can take the values of 0 or 1
R is hydrogen, methyl or ethyl.
and stereoisomers or hydrates thereof.

2. A compound according to claim 1 wherein R is selected from the group consisting of hydrogen or methyl and stereoisomers or hydrates thereof.

3. A compound according to claim 2 wherein m has a value of 0 and R is hydrogen or methyl and stereoisomers or hydrates thereof.

4. A compound according to claim 3 wherein n has a value of 1 and R is hydrogen and hydrates thereof.

5. A compound as defined in anyone of claims 1 to 4 for use as a medicament.

6. A compound as defined in anyone of claims 1 to 4 for use in the treatment and/or prophylaxis of a cartilage and/or bone disease and/or conditions resulting in a dysregulation of cartilage and/or bone metabolism in a mammal, such as, e. g. , a human female or male adult, adolescent or a child, such as, e. g. , osteoporosis, osteoarthritis, osteopetrosis, osteopenia and Paget's disease, hypercalcemia of malignancy, periodontal disease, hyperparathyroidism, periarticular erosions in rheumatoid arthritis, osteodystrophy, myositis ossificans, Bechterew's disease, malignant hypercalcemia, osteolytic lesions produced by bone metastasis, bone pain due to bone metastasis, bone loss due to sex steroid hormone deficiency, bone abnormalities due to steroid hormone treatment, bone abnormalities caused by cancer therapeutics, osteomalacia, Bechet's disease, hyperostosis, metastatic bone disease, immobilization-induced osteopenia or osteoporosis, or glucocorticoid-induced osteopenia or osteoporosis, osteoporosis pseudoglioma syndrome, idiopathic juvenile osteoporosis, for the improvement of fracture healing after traumatic or atraumatic fracture, and for the maintenance or increase of energy level, for building up or strengthening muscle tissues and for weight gain.

7. A compound for use according to claim 6 wherein the bone disease is osteoporosis.

8. A pharmaceutical composition comprising a compound as defined in anyone of claims 1 to 4 and a pharmaceutically acceptable carrier.

9. A pharmaceutical composition according to claim 8 which further comprises one or more drugs selected from the group consisting of vitamin D; estrogen analogs useful in estrogen replacement therapy such as estradiol and conjugate equine estrogens (CEE); parathyroid hormone and its analogs such as Teriparatide and PTH-131A; selective estrogen receptor modulators (SERMs) such as Raloxifene, Lasofoxifene, Arzoxifene and Bazedoxifene; selective tissue estrogenic activity regulators such as Tibolone; bisphophonates such as Zoledronate, Risedronate, Ibandronate, Alendronate, Olpadronate, Neridronate, Pamidronate, Etidronate, Clodronate, Tiludronate; Calcitonines such as salcitonine; sodium fluoride; cathepsin K inhibitors such as Odanacatib and ONO-5334; antagonists for the receptor activator of nuclear factor kappa B ligand (RANKL) such as Osteoprotegerin and monoclonal antibodies targeting RANKL such as Denosumab; 3-hydroxy-3-methyl-glutaryl-CoA (HMG-CoA) reductase inhibitors such as Atorvastatin, Cerivastatin, Fluvastatin, Lovastatin, Mevastatin, Pitavastatin, Pravastatin, Rosuvastatin, Simvastatin and other strontium salts such as strontium citrate, strontium malonate and strontium ranelate.
